Europäisches Patentamt

(19) European Patent Office

Office européen des brevets

(11) Publication number: **0 061 701**
**B1**

(12) **EUROPEAN PATENT SPECIFICATION**

(45) Date of publication of patent specification: **16.07.86**

(21) Application number: **82102408.0**

(22) Date of filing: **23.03.82**

(51) Int. Cl.⁴: **A 61 K 7/035,** A 61 K 7/32,
A 61 K 7/48

(54) A solid entrapped emollient-moisturizer composition and the use thereof.

(30) Priority: **23.03.81 US 246663**

(43) Date of publication of application:
**06.10.82 Bulletin 82/40**

(45) Publication of the grant of the patent:
**16.07.86 Bulletin 86/29**

(84) Designated Contracting States:
**CH DE FR GB IT LI**

(56) References cited:
DE-A-2 705 218
FR-A-2 185 415
GB-A- 774 029
GB-A-1 261 435

COSMETICS & TOILETRIES, vol. 96, April 1981,
pages 47-50, Presented in part at the S.C.C.
Annual Meeting, 12th December 1980, ref. no.
1, (USA); A.V. CALOGERO: "Polymer
entrapment systems in makeup applications"

M.S.BALSAM et al.: "Cosmetics Science and
Technology", 2nd Edition, vol. 1, 1972, pages
34-36, John Wiley & Sons, New York (USA);

(73) Proprietor: **Wickhen Products, Inc.**
**Big Pond Road**
**Huguenot New York 12746 (US)**

(72) Inventor: **Abrutyn, Eric Steven**
**9 Westminster Drive**
**Middletown, New York, N.Y. (US)**

(74) Representative: **Hansen, Bernd, Dr.rer.nat. et al**
**Hoffmann, Eitle & Partner Patentanwälte**
**Arabellastrasse 4**
**D-8000 München 81 (DE)**

(56) References cited:
CHEMICAL ABSTRACTS, vol. 92, no. 24, 16th
June 1980, page 320, col. 1-2, no. 203410f,
Columbus Ohio (USA); A.V. CALOGERO:
"Branched chain esters & Their applications in
haircare products"

JOURN. SOC. COSMET. CHEM., vol. 28, 1977,
pages 377-393; J.P. GUILLOT et al.: "Safety
evaluation of cosmetic raw materials"

N.F.ESTRIN et al.: "CFTA Cosmetic Ingredient
Dictionary", 3rd Edition, 1982, The Cosmetic,
Toiletry and Fragrance Association, Inc.,
Washington (USA);

Courier Press, Leamington Spa, England.

## Description

This invention relates to a powdery, solid free flowing entrapped emollient and/or moisturizer polymer composition.

The art is replete with attempts to render functional materials amenable to release on demand through encapsulation. Encapsulation confines materials in discrete units as the result of coating particles of the material with an encapsulant. The coating or wall material used in encapsulation includes natural or synthetic polymers which permit release of the functional material by fracture, degradation or diffusion.

DE—A—2 705 218 discloses polymer compositions of specific hydrophylic monomers and a volatile active ingredient. The volatile active ingredient is at least partly dissolved in the polymer mass and a solubilizer must be present which is compatible with the polymer and which is at least with a part of the active ingredient more compatible then with the polymer. Thus, hydrophylic polymers are used to hold the functional material. Furthermore, cosmetic preparations containing a hydrophylic polymer are described in GB—A—1 261 435.

The object of the present invention is to provide a unit combination of polymeric encapsulance and entrapped a mollients and/or moisturizers which result in compositions which can be incorporated into a variety of products such as cosmetics and health care products at levels which heretofore have been impossible to achieve.

This object is solved by a powdery, solid free flowing entrapped emollient and/or moisturizer polymer composition, the monomers of which having been polymerized in situ which is characterized in that it comprises from 5 to 95 weight percent of a crosslinked syneresis free hydrophobic polymer lattice into which are incorporated as an emollient and/or moisturizer from 95 to 5 weight percent of a compound selected from a straight, branched or cyclic alcohol containing 1 to 30 carbon atoms, a straight, branched or cyclic carboxylic acid containing 1 to 30 carbon atoms, an ester containing a $C_1$ to $C_{30}$ carboxylic acid esterified with a $C_1$ to $C_{30}$ alcohol, an alcohol ether containing 1 to 30 carbon atoms, an alkane of the formula $H—(CH_2)_n—H$ wherein n is 5 to 30, lanolin and its derivates, and a siloxane.

The solid, entrapped emollient and/or moisturizer composition of the invention may also contain from 10 to 90 weight percent fragrance, 0.1 to 10 weight percent of an organic soluble dye, 0.1 to 10 weight percent of a lake or 0.1 to 10 weight percent of a pigment.

It has now discovered that a wide variety of materials commonly referred to as emollients or moisturizers which are either liquids or solids can be converted to free-flowing powders or beads by entrapment of the materials in a hydrophobic polymeric lattice. The polymeric lattice functions to hold and protect the entrapped material, probably through sorption or swelling, and is capable of providing availability of the entrapped material by a variety of mechanisms including pressure, diffusion and extraction. Significantly, when the products of this invention are incorporated in cosmetic and toiletry products the polymeric lattice itself contributes to the beneficial effects to be imparted by application of the entrapped emollient-moisturizer material.

While this invention relates primarily to entrapment of emollient and/or moisturizers within the polymeric lattice, those skilled in the art will recognize that a wide variety of functional materials can be entrapped within the polymeric lattice. For example, the invention contemplates that a wide variety of water insoluble organic liquids and solids may be incorporated within the lattice matrix. In fact, any functional material which will not react with the polymer system comprising the polymeric lattice matrix can be entrapped within the polymeric lattice.

The application will discuss the invention as it relates specifically to emollient and/or moisturizer entrapped products. The terms "emollient" and "moisturizer" include materials having properties defined for those terms in the text and articles:

M. G. de Navarre, *The Chemistry and Manufacture of Cosmetics*, Vol. 3, 2nd Ed. 1975, Chapter 9.

"Moisturization; A Systematic Approach"—L. J. Murphy *Cosmetics and Toiletries*, Vol. 93 (March, 1978) p. 31

"Mineral Oil and Petrolatum; Reliable Moisturizers" by F. Tranner and G. Berube—*Cosmetics and Toiletries*, Vol. 93 (March, 1978) p. 81

The solid, entrapped emollient and/or moisturizer compositions of this invention are prepared by combining a functional crosslinking monomer, a monofunctional monomer and the functional material to be entrapped under such conditions as to initiate polymerization. As used herein, the term "functional crosslinking monomer" is meant to include di or polyfunctional monomers having two or more polymerizable double bonds, while the term "monofunctional monomer" is meant to include a polymerizable monomer having one double bond. Functional crosslinking monomers useful in the invention may be a polyunsaturated monomer selected from a mono-, di- or polyester of a mono-, di- or polyhydric alcohol and an α-β unsaturated carboxylic acid, polyunsaturated polyvinyl ether of a polyhydric alcohol; mono- or poly unsaturated amides and cycloaliphatic esters of α-β unsaturated carboxylic acids. Examples of such functional crosslinking monomers include polyethylene glycols having a molecular weight up to about 5000 including methylene glycol dimethacrylate, diethylene glycol dimethacrylate, triethylene glycol dimethacrylate, tetraethylene glycol dimethacrylate and trimethylol propane ethoxylated triacrylate, available under the trademark CHEMLINK® 176, ditrimethylol propane dimethacrylate; propylene, dipropylene and higher propylene glycols having a molecular weight up to about 5000 including polyethylene glycol dimethacrylate, 1,3 butylene glycol dimethacrylate, 1,4 butanediol dimethacrylate, 1,6 hexanediol dimethacrylate, neopentyl glycol dimethacrylate, pentaerythritol dimethacrylate, dipentaerythritol dimethacrylate, bisphenol A dimethacrylate, divinyl (trivinyl) benzene, divinyl trivinyl toluene, triallyl maleate, triallyl phosphate, diallyl maleate, diallyl itaconate, and allyl methacrylate.

The monofunctional monomers include alkyl methacrylates and acrylates having straight or branched chain alkyl groups with 1 to 30 carbon atoms, preferably 5 to 18 carbon atoms. Preferred monofunctional monomers include lauryl methacrylate, 2-ethylhexyl methacrylate, isodecyl methacrylate, stearyl methacrylate, hydroxyethyl methacrylate, hydroxypropyl methacrylate, diacetone acrylamide, phenoxyethyl methacrylate, tetrahydrofurfuryl methacrylate and methoxyethyl methacrylate.

The functional materials to be entrapped within the novel polymeric matrix of this invention are selected from materials commonly referred to as emollients and moisturizers, materials which are normally either liquids or solids. Functional materials such as perfumes, fragrances and flavors also may be combined with emollients and/or moisturizers and entrapped within the novel polymeric matrix of this invention. Examples of emollients and moisturizers which may be entrapped within the polymeric matrix of this invention include straight, branched or cyclic alcohols containing 1 to 30 carbon atoms; straight, branched, or cyclic carboxylic acids containing 1 to 30 carbon atoms; esters containing $C_1$ to $C_{30}$ carboxylic acids esterified with $C_1$ to $C_{30}$ alcohols; alcohol ether containing 1 to 30 carbon atoms; alkanes of the formula $H—(CH_2)_n—H$, wherein n is 5 to 30; and siloxanes. Examples of such functional materials include 2-ethylhexyl oxystearate available commercially as WICKENOL® 171; arachidyl propionate available commercially as WAXENOL® 801, 2-ethylhexyl adipate available commercially as WICKENOL® 158; isopropyl myristate available commercially as WICKENOL® 101; ethanol; stearyl alcohol; propylene glycol; propionic acid; stearic acid; polyoxypropylene cetyl alcohol, available commercially as WICKENOL® 707; polyoxypropylene lanolin alcohol available commercially as WICKENOL® 727; Carbowax® 300; petroleum jelly; mineral oil; aliphatic hydrocarbons such as mineral spirits; lanolin and lanolin derivatives such as acetylated lanolin and isopropyl lanolate; hexamethyl disiloxane, available commercially as DOW® Q2-1096; cyclic polydimethyl siloxane, available commercially as DOW® 344 and 345; and linear polydimethyl siloxane, available commercially as DOW® 200; poly phenyl methyl siloxane, available commercially as DOW® 556; and poly dimethyl/trimethyl siloxane. Other phenyl, ethyl and vinyl substituted polysilanes may also be included in the products of this invention.

The crosslinking monomer, monofunctional monomer and functional material are combined in a ratio such that the novel entrapped composition of this invention comprises from 5 to 95 weight percent of a crosslinked polymer matrix and from 95 to 5 weight percent of the functional material. The ratio of crosslinking monomer to monofunctional monomer in the crosslinked polymer matrix can vary within the range of 99:1 to 1:99. While not restricting the invention to any precise composition, in a typical product of this invention, the crosslinking monomer, monofunctional monomer and functional material are combined in a ratio such that the novel crosslinked polymer matrix comprises from 60 to 80 weight percent of the functional monomer.

The crosslinked polymer matrix containing the entrapped functional material results from in situ polymerization of the monomer mixture containing the functional material desired to be entrapped. Generally, this results from mixing the crosslinking monomer and monofunctional monomer, combining the functional material therewith to form a uniform mixture, and inducing polymerization. Polymerization may be induced by conventional initiators such as peroxides and the like, or by irradiation or redox systems. Polymerization usually occurs at temperatures between about 0° to 120°C, preferably about 80°C. The time and temperature of polymerization may be varied in accordance with nature of the functional material, its concentration, and the attributes of the desired entrapped system.

The physical properties of the entrapped functional materials may be influenced by several factors such as the precise combination of crosslinking monomer and monofunctional monomer selected, the ratio in which these two components are combined with one another and with the functional material. Accordingly, the entrapped materials of this invention which exist in the form of discrete, free-flowing powders or beads may be hard and have the ability to withstand rather substantial shearing or the powders or beads may be soft, in which form they liquify with minimal pressure. In general, the greater the ratio of crosslinked polymer matrix to functional material, the harder the entrapped material. The entrapped functional material ranges in particle size from about 0.001 millimeters to about 3 millimeters.

A simple test has been developed to enable prediction with reasonable accuracy whether or not a particular combination of crosslinking monomer, monofunctional monomer and functional material will polymerize to form the entrapped functional material of this invention. According to this test, approximately equal quantities of crosslinking monomer, monofunctional monomer and functional material are combined in a test tube, and polymerized. If the resultant polymerized product is turbid or cloudy, a heterogeneous macroporous structure has formed which is a positive indication that the components tested can be combined in a ratio such that subsequent polymerization will result in the products of this invention. There are exceptions to this rule, in that certain combinations of materials may result in production of a clear polymer. If, however, the polymer is extracted from the reaction mixture and is determined to be cloudy or turbid, indicating a heterogeneous, macroporous structure, a positive test has again occurred. After a positive test, i.e., an initial turbid or cloudy appearance on polymerization of the test tube size sample, further tests are conducted by varying the ratio of monomers to functional material to determine those ranges in which discrete particles, and not clumps or masses, are obtained on polymerization. With the foregoing test in mind, and recognizing the need to obtain discrete particles and not clumped or massed polymers, it will be appreciated that those skilled in the art can select appropriate crosslinking monomers, monofunctional monomers and the ratio in which these materials are to be combined to obtain the entrapped materials of this invention.

The novel entrapped functional materials of this invention are versatile products having application in many and varied types of products. As stated previously, liquid and solid emollients and moisturizers form entrapped products suitable for incorporation in a wide variety of cosmetic, beauty and health care products. Insecticides, disinfectants, sun screens, flavors, pigments and perfumes may also be used as additional materials in the entrapped system of this invention. Of course, the primary advantage of formation of the novel entrapped functional materials of this invention is the conversion of liquid or solid emollients and moisturizers into powdery, free-flowing materials through incorporation in a syneresis free hydrophobic polymeric lattice having the ability to hold the functional materials for controlled application on demand. Other advantages of the entrapped functional materials of this invention include the ability to convert the solid and liquid functional materials into free-flowing discrete particles ranging in size from fine powders to rather large beads. Still another advantage of this invention lies in the fact that the polymer matrix itself provides desirable attributes on incorporation of the entrapped functional materials in cosmetics or toiletry preparations.

The entrapped functional materials of this invention are easy to handle, convenient to store, and are prepared by relatively non-complex procedures. Entrapment of the functional materials within the crosslinked polymer matrix or lattice protects the functional materials from many things, such as the environment, excessive volatilization, and from ultraviolet light. The functional materials are available from their entrapped state within the microscopic lattice by pressure, and diffusion due to temperature and humidity changes, or by extraction. Also, it has been found that the desirable characteristics of the entrapped functional materials, i.e., emollients and moisturizers, are enhanced by the polymer matrix itself. The polymer matrix or lattice provides a continuous film when applied to the skin, and the total effect of the compositions of this invention is to extend the emollient-moisturizer effect of the entrapped materials.

A decided advantage of the entrapped functional materials of this invention results from their capability of incorporating substantially high levels of functional material in a desired product than is possible through incorporation of the raw functional material. For example, it is known that an emollient such as 2-ethylhexyl oxystearate (WICKENOL® 171) provides improved quantities of skin feel and moisturizing capability to toilet soap. However, it is not possible to incorporate more than about 2—5% of such an emollient in conventional toilet soap formulations without seriously detracting from the foaming characteristics of the soap. If the same functional material is formulated in the entrapped microscopic polymeric lattice of this invention, substantially higher concentrations of the functional material, up to as much as 20%, by weight, thereof, may be incorporated in the toilet soap formulation where it serves to enhance the feel and moisturizing properties of the soap without any deleterious effect on the foaming and esthetic properties of the soap. The polymer portion of the complex also improves the mechanical properties of the soap.

Another area of important application of the novel entrapped functional materials of this invention is in molded wax and/or oil base sticks of the type typically used for antiperspirants, deodorants, lipsticks, sunscreens, insect repellants and colognes. Typically, such stick type products balance many ingredients to give a desired appearance and function, but the optimal solid wax-oil base stick seems to elude cosmetic formulators as problems of shrinkage, variable rate of deposition on the skin, tackiness, and the like, continue to plague such products. The entrapped functional materials of this invention offer significant advantages for such stick type products as they make it possible to substantially reduce the bodying agents such as natural vegetable or insect waxes typically present in such stick products. This results from the fact that the polymeric lattice entrapping the functional material of this invention enhances rigidity and strength of the stick while permitting the entrapped functional material to produce their desired effect as they are made available from their entrapped state.

The entrapped functional materials of this invention are free flowing powders which are easy to handle and convenient to store. The functional materials are made available when applied to the skin either directly or as a component of a cosmetic or toiletry product through what is theorized to be a unique and novel mechanism. It is thought that the functional material acts in a manner analogous to that of a plasticizer when incorporated in the polymerized hydrophobic matrix, and that when the entrapped functional material is applied to the body in a cosmetic or toiletry product it is made available as the result of rubbing and spreading in the form of a continuous uniform film protected within a hydrophobic envelope.

While it will be appreciated by those skilled in the art that there are many variations in procedure and components, this invention may illustrated by the following examples:

Example 1

7 grams of 2-ethylhexyl oxystearate (WICKENOL® 171) was mixed with 1.5 grams of ethylene glycol dimethacrylate and 1.5 grams of lauryl methacrylate in a glass test tube. The solution was deaerated for five (5) minutes and 0.1 ml of t-butyl peroctoate was added and mixed while heating to 80°C in an oil bath. After 20 minutes, the contents solidified; and the mixture was maintained at about 80°C for an additional hour to assure full polymerization. A semi-soft, heterogeneous white opaque polymer mass resulted containing the entrapped ester.

The following examples demonstrate initial screening of crosslinking monomer, monofunctional monomer and functional material to determine whether or not the combination thereof will form the novel entrapped products of the invention. In each test the components are combined in a test tube and polymerization initiated and completed. Formation of an opaque polymer mass in the test tube scale test indicates that the components can be combined in large scale polymerization to form the entrapped functional materials of this invention.

Example 2

Following the procedure of Example 1, the crosslinking monomers tetraethylene glycol dimethacrylate, trimethylolpropane trimethylacrylate, trimethylol-propane ethoxy triacrylate, and allyl methacrylate were polymerized in the presence of 70%, by weight, of 2-ethylhexyl oxystearate and 15%, by weight, of lauryl methacrylate. In each case a semi-soft, white opaque polymer mass resulted, indicating suitability for formation of the entrapped product of this invention.

Example 3

Following the procedure of Example 1, test tube polymerization was completed varying the types of monomer constituents and their ratios, and the quantity and type of functional material to be entrapped. In each instance, t-butyl peroctoate was used to initiate polymerization at a constant level of 3 weight percent, based on the weight of the combined content of monomers and functional material. The components, quantity and test tube results are set forth in Table 1. The following abbreviations are used in Table 1:

TEGDM Tetraethylene glycol dimethacrylate
TMPTM Trimethyl propane trimethacrylate
EGDM Ethylene glycol dimethacrylate
TPETM Trimethylol propane ethoxylate trimethacrylate
LMA Lauryl methacrylate
IMA Isodecyl methacrylate
HMA Hydroxyethyl methacrylate
DAA Diacetone acrylamide
PMA Phenoxyethyl methacrylate
MEMA Methoxy ethyl methacrylate

TABLE I

| Test No. | Cross-linking monomer | Weight % | Mono functional monomer | Weight % | Material entrapped | Weight % | Appearance in test tube |
|---|---|---|---|---|---|---|---|
| 1 | TEGDM | 67.5 | LMA | 22.5 | 2-Ethylhexyl oxy-stearate (WICKENOL® 171) | 10 | Hard-powdery, white opaque polymer mass |
| 2 | TMPTM | 45 | IMA | 45 | Arachidyl pro-pionate (WAXENOL® 801) | 10 | Semi-hard, off-white opaque |
| 3 | TMPTM | 12 | IMA | 3 | Arachidyl pro-pionate (WAXENOL® 801) | 85 | Semi-soft, off-white opaque |
| 4 | EGDM | 18.7 | SMA | 6.3 | Di(Ethylhexyl) adipate (WAXENOL® 158) | 75 | Semi-soft, white opaque |
| 5 | EGDM | 30 | HMA | 10.3 | Isopropyl Myristate (WICKENOL® 101) | 60 | Semi-soft, white opaque |
| 6 | EGDM | 30 | LMA | 10 | Ethanol | 60 | Hard-powdery, white opaque |

TABLE I (continued)

| Test No. | Cross-linking monomer | Weight % | Mono functional monomer | Weight % | Material entrapped | Weight % | Appearance in test tube |
|---|---|---|---|---|---|---|---|
| 7 | TEGDM | 67.5 | SMA | 22.5 | Stearyl alcohol | 10 | Very hard, white opaque |
| 8 | TEGDM | 15 | SMA | 5 | Stearyl alcohol | 80 | Hard-powdery white opaque |
| 9 | EGDM | 67.5 | DAA | 22.5 | Propylene glycol | 10 | Very hard off-white opaque |
| 10 | EGDM | 15 | DAA | 5 | Propylene glycol | 80 | Semi-soft, off-white opaque |
| 11 | EGDM | 60 | LMA | 30 | Propionic acid | 10 | Very hard, white opaque |
| 12 | EGDM | 15 | LMA | 5 | Propionic acid | 80 | Semi-hard white opaque |
| 13 | TEGDM | 45 | SMA | 45 | Stearic acid | 10 | Very hard, white opaque |
| 14 | TEGDM | 10 | SMA | 10 | Stearic acid | 80 | Semi-hard white opaque |
| 15 | EGDM | 67.5 | SMA | 22.5 | Polyoxy propylene (30 moles) cetyl alcohol (WICKENOL® 707) | 10 | Very hard, white opaque |
| 16 | EGDM | 15 | SMA | 5 | | 80 | Semi-soft, white opaque |
| 17 | EGDM | 60 | DAA | 30 | Polyoxy propylene (30 moles lanolin) (WICKENOL® 727) | 10 | Very hard, white opaque |
| 18 | EGDM | 15 | DAA | 5 | | 80 | Semi-soft, yellowish, opaque |
| 19 | TEGDM | 67.5 | LMA | 22.5 | Carbowax® 300 | 10 | Hard and clear |
| 20 | TEGDM | 13 | LMA | 7 | | 80 | Semi-soft, white opaque |
| 21 | TPETM | 54 | PMA | 36 | Mineral oil | 10 | Hard-powdery, white opaque |
| 22 | TPETM | 15 | PMA | 15 | Mineral oil | 70 | Semi-soft, white opaque |
| 23 | TMPTM | 45 | MEMA | 45 | Petroleum jelly | 10 | Semi-soft, white opaque |
| 24 | TMPTM | 15 | MEMA | 5 | Petroleum jelly | 80 | Semi-soft, white opaque |
| 25 | EGDM | 45 | LMA | 45 | Mineral spirits | 10 | Hard powdery, white opaque |
| 26 | EGDM | 18.8 | LMA | 6.2 | Mineral spirits | 75 | Semi-hard, white opaque |

## 0 061 701

TABLE I (continued)

| Test No. | Cross-linking monomer | Weight % | Mono functional monomer | Weight % | Material entrapped | Weight % | Appearance in test tube |
|---|---|---|---|---|---|---|---|
| 27 | TEGDM | 12.5 | LMA | 12.5 | Lanolin | 75 | Semi-soft, yellow opaque |
| 28 | EGDM | 60 | SMA | 30 | Poly-Hexa-methyl disiloxane (Dow® Q2-1096) | 10 | Very hard, white opaque |
| 29 | EGDM | 15 | SMA | 5 | | 80 | Hard, powdery, white opaque |
| 30 | EGDM | 60 | LMA | 30 | Poly-dimethyl (cyclic) siloxane (Dow® 344 & 345) | 10 | Hard, powdery, white opaque |
| 31 | EGDM | 22.5 | LMA | 7.5 | | 70 | Hard, powdery, white opaque |
| 32 | EGDM | 45 | DAA | 45 | Polydimethyl (Linear) Siloxane (Dow® 200) | 10 | Very hard, white opaque |
| 33 | EGDM | 10 | DAA | 10 | | 80 | Semi-hard, white opaque |

The following examples demonstrate formation of the entrapped materials of this invention.

Example 4

1.20 grams of polyvinyl pyrrolidone having a K value of about 80 to 100 was dissolved in 1500 ml of water in a 2000 ml three necked resin flask equipped with a stirrer, thermometer and nitrogen purge. A solution of 335 grams of 2-ethylhexyl oxystearate (WICKENOL® 171), 132 grams ethylene glycol dimethacrylate, 33 grams 2-ethylhexyl methacrylate and 5 ml t-butyl peroctoate was bubbled with nitrogen for 5 minutes. The resultant monomer mix was slowly added to the stirred aqueous solution of polyvinyl pyrrolidone at 22°C under nitrogen. The temperature was raised to 80°C with constant agitation and held until polymerization started in approximately 15 minutes, and maintained at 80°C for an additional 2 hours to complete reaction. Semi-soft, white opaque beads were collected by filtering off the supernatant liquid and dried to remove any excess water. The beads weighed 450 g for a yield of 90%, and were 0.25 to 0.5 mm in diameter. Other protective colloids such as starch, polyvinyl alcohol, carboxymethyl cellulose, methyl cellulose, or inorganic systems such as alkali metal hydroxides may be used in place of the polyvinyl pyrrolidone suspending medium.

Example 5

The procedure of Example 4 was repeated except that in each case 337.5 g arachidyl propionate (WAXENOL® 801), or 337.5 g mineral oil, or 350 g cyclic polydimethyl siloxane (Dow® 345), or 350 g petroleum distillate (150 to 160°C, boiling point), or 325 g petroleum jelly, or 350 g isopropyl isostearate (WICKENOL® 131), or 375 g di(2 ethylhexyl) adipate (WICKENOL® 158), were substituted for 2-ethylhexyl oxystearate. In each case, semi-soft, white opaque beads were collected in good yield. These beads may be incorporated in cosmetic or toiletry products where they demonstrate their desired effect by making the entrapped emollient-moisturizer available for application to the skin. The particle size of the resultant bead in each case was between 0.25 to 0.5 mm in diameter, the precise particle size varying somewhat in the degree and rate of agitation during polymerization and the rates of the components to the water in which the polymerization system was suspended.

The following examples demonstrate cosmetic or toiletry compositions in which the entrapped

7

functional materials of this invention have been incorporated.

### Example 6
### Translucent pressed powder

| | |
|---|---|
| Talc | 77.64 |
| Kaolin | 14.00 |
| 75% Arachidyl propionate entrapped bead of Example 5 | 5.00 |
| Magnesium carbonate | 2.00 |
| Colorants | 0.31 |
| Methyl paraben | 0.10 |
| Propyl paraben | 0.10 |
| Germall® 115 | 0.10 |
| Fragrance | 0.75 |
| | 100.00 |

The components were combined in accordance with conventional formulation techniques. The entrapped emollients (Example 5 product) provided the pressed powder with desired emollient properties and application of the product to the body makes the emollient available by rubbing. The pressed powder was remarkably resistant to breakage, crumbling and glazing.

### Example 7
### Milled toilet soap

| | |
|---|---|
| Toilet soap base of tallow and coconut[1] | 89.00 |
| 2-ethylhexyl oxystearate entrapped bead of Example 4 | 10.00 |
| Fragrance | 1.00 |
| | 100.00 |

[1]Duveen Soap Corporation, 154 Morgan Avenue, Brooklyn, New York.

The components were combined in accordance with conventional formulation techniques. The entrapped emollient (Example 4) provided the soap with desired emollient properties. In addition, the physical attributes of the soap were enhanced, rendering it more resistant to cracking in use and less brittle. The soap had excellent lathering properties.

### Example 8
### Body powder

| | |
|---|---|
| Talc | 84.5 |
| Fragrance | 0.5 |
| 2-ethylhexyl oxystearate entrapped bead of Example 4 | 10.00 |
| Syloid #74 | 5.00 |
| | 100.00 |

The components were combined in accordance with conventional formulation techniques. The entrapped emollient (Example 4) provided the body powder with desired emollient properties. In addition, the physical properties of the body powder were enhanced, providing increased adhesion to the body.

### Example 9
### Antiperspirant stick

| | |
|---|---|
| Phase A | |
| Stearyl alcohol | 25.0 |
| Synthetic Beeswax Flakes[a] WAXENOL® 821 | 10.0 |
| Myristyl Myristate[a] WAXENOL® 810 | 25.0 |
| Propylene glycol stearate | 5.0 |
| Phase B | |
| Aluminium chlorhydrate[a] WICKENOL® CPS 325 | 25.0 |
| Phase C | |
| 2-Ethylhexyl oxystearate entrapped bead of Example 4 | 5.0 |
| Di-octyl adipate entrapped bead of Example 5 | 5.00 |
| | 100.00 |

[a]Wickhen Products, Inc., Huguenot, New York 12746.

The antiperspirant stick formulations are prepared by heating the components of Phase A to 65—70°C until melted, adding the component of Phase B without further heating and with constant and continuous agitation followed by slow addition of the components of Phase C with constant agitation until a uniform mixture is obtained. The mixture is then cooled somewhat and poured into molds at temperatures of from about 50 to 55°C. The antiperspirant stick has enhanced rigidity and strength and desired emollient properties without tackiness.

**Claims**

1. A powdery, solid free flowing entrapped emollient and/or moisturizer polymer composition, the monomers of which having been polymerized in situ, characterized in that it comprises from 5 to 95 weight percent of a crosslinked syneresis free hydrophobic polymer lattice into which are incorporated as an emollient and/or moisturizer from 95 to 5 weight percent of a compound selected from a straight, branched or cyclic alcohol containing 1 to 30 carbon atoms, a straight, branched or cyclic carboxylic acid containing 1 to 30 carbon atoms, an ester containing a $C_1$ to $C_{30}$ carboxylic acid esterified with a $C_1$ to $C_{30}$ alcohol, an alcohol ether containing 1 to 30 carbon atoms, an alkane of the formula H—$(CH_2)_n$—H wherein n is 5 to 30, lanolin and its derivates, and a siloxane.

2. The solid, entrapped emollient moisturizer composition of claim 1 wherein said crosslinked syneresis free hydrophobic polymer matrix is based on a di or polyfunctional crosslinking monomer and a hydrophobic monounsaturated monomer.

3. The solid, entrapped emollient and/or moisturizer composition of claim 2 wherein said crosslinking monomer is a polyunsaturated monomer selected from a mono-, di- or polyester of mono-, di- or polyhydric alcohol and an α-β unsaturated carboxylic acid, polyunsaturated polyvinyl ether of a polyhydric alcohol, mono- or polyunsaturated amides and cycloaliphatic esters of α-β unsaturated carboxylic acids.

4. The solid, entrapped emollient and/or moisturizer composition of Claim 1 wherein said emollient and/or moisturizer contains from 10 to 90 weight percent fragrance.

5. The solid, entrapped emollient and/or moisturizer composition of Claim 1 wherein said emollient moisturizer contains from 0.1 to 10 weight percent of an oil soluble dye.

6. The solid, entrapped emollient and/or moisturizer composition of Claim 1 wherein said emollient-moisturizer contains from 0.1 to 10 weight percent of a lake.

7. The solid, entrapped emollient and/or moisturizer composition of Claim 1 wherein said emollient-moisturizer contains from 0.1 to 10 weight percent of a pigment.

8. The solid, entrapped emollient and/or moisturizer composition of Claim 1 wherein said emollient-moisturizer is 2-ethylhexyloxystearate.

9. The solid, entrapped emollient and/or moisturizer composition of Claim 1 wherein said emollient-moisturizer is a mineral oil.

10. The solid, entrapped emollient and/or moisturizer composition of Claim 1 wherein said emollient-moisturizer is arachidyl propionate.

11. The solid, entrapped emollient and/or moisturizer composition of Claim 1 wherein said emollient-moisturizer is petroleum jelly.

12. The solid, entrapped emollient and/or moisturizer composition of Claim 1 wherein said emollient-moisturizer is a siloxane.

13. The use of the composition according to Claims 1—12 for cosmetic and toiletry composition.

**Patentansprüche**

1. Pulverförmige, feste, freifliessende, eingeschlossene Erweichungsmittel- und/oder Befeuchtungsmittel-Polymerzusammensetzung, wobei die Monomeren in situ polymerisiert worden sind, dadurch gekennzeichnet, dass sie 5 bis 95 Gew.% eines vernetzten synäresefreien, hydrophoben Polymergitters umfasst, in welche als ein Erweichungsmittel und/oder Befeuchtungsmittel 95 bis 5 Gew.% einer Verbindung, ausgewählt aus einem geradkettigen, verzweigten oder cyclischen Alkohol, enthaltend 1 bis 30 Kohlenstoffatome, einer geradkettigen, verzweigten oder cyclischen Carboxylsäure, enthaltend 1 bis 30 Kohlenstoffatome, einem Ester, enthaltend eine $C_{1-30}$-Carboxylsäure, die mit einem $C_{1-30}$-Alkohol verestert ist, einem Alkoholether, enthaltend 1 bis 30 Kohlenstoffatome, einem Alkan der Formel H—$(CH_2)_n$—H, worin n 5 bis 30 ist, Lanolin oder dessen Derivaten und einem Siloxan, inkorporiert sind.

2. Feste, eingeschlossene Erweichungsmittel-Befeuchtungsmittel-Zusammensetzung gemäss Anspruch 1, worin die vernetzte synäresefreie, hydrophobe Polymermatrix auf einem di- oder polyfunktionellen Vernetzungsmonomer und einem hydrophoben, monoungesättigten Monomer aufgebaut ist.

3. Feste, eingeschlossene Erweichungsmittel- und/oder Befeuchtungsmittel-Zusammensetzung gemäss Anspruch 2, worin das vernetzende Monomere ein polyungssättigtes Monomer, ausgewählt aus einem Mono-, Di- oder Polyester eines ein-, zwei- oder mehrwertigen Alkohols und einer α-β-ungesättigten Carboxylsäure, einem polyungesättigten Polyvinylether eines mehrwertigen Alkohols, von mono- oder polyungesättigten Amiden und cycloaliphatischen Estern von α-β-ungesättigten Carboxylsäuren ausgewählt ist.

4. Feste, eingeschlossene Erweichungsmittel- und/oder Befeuchtungsmittel-Zusammensetzung gemäss Anspruch 1, worin das Erweichungsmittel und/oder Befeuchtungsmittel 10 bis 90 Gew.% Riechstoff enthält.

5. Feste, eingeschlossene Erweichungsmittel- und/oder Befeuchtungsmittel-Zusammensetzung gemäss Anspruch 1, worin das Erweichungsmittel-Befeuchtungsmittel 0,1 bis 10 Gew.% eines öllöslichen Farbstoffs enthält.

6. Feste, eingeschlossene Erweichungsmittel- und/oder Befeuchtungsmittel-Zusammensetzung gemäss Anspruch 1, worin das Erweichungsmittel-Befeuchtungsmittel 0,1 bis 10 Gew.% eines Lacks enthält.

7. Feste, eingeschlossene Erweichungsmittel- und/oder Befeuchtungsmittel-Zusammensetzung gemäss Anspruch 1, worin das Erweichungsmittel-Befeuchtungsmittel 0,1 bis 10 Gew.% an Pigment enthält.

8. Feste, eingeschlossene Erweichungsmittel- und/oder Befeuchtungsmittel-Zusammensetzung gemäss Anspruch 1, worin das Erweichungsmittel-Befeuchtungsmittel 2-Ethylhexyloxystearat ist.

9. Feste, eingeschlossene Erweichungsmittel- und/oder Befeuchtungsmittel-Zusammensetzung gemäss Anspruch 1, worin das Erweichungsmittel-Befeuchtungsmittel ein Mineralöl ist.

10. Feste, eingeschlossene Erweichungsmittel- und/oder Befeuchtungsmittel-Zusammensetzung gemäss Anspruch 1, worin das Erweichungsmittel-Befeuchtungsmittel Arachidylpropionat ist.

11. Feste, eingeschlossene Erweichungsmittel- und/oder Befeuchtungsmittel-Zusammensetzung gemäss Anspruch 1, worin das Erweichungsmittel-Befeuchtungsmittel ein Petrolgel ist.

12. Feste, eingeschlossene Erweichungsmittel- und/oder Befeuchtungsmittel-Zusammensetzung gemäss Anspruch 1, worin das Erweichungsmittel-Befeuchtungsmittel ein Siloxan ist.

13. Verwendung der Zusammensetzung gemäss Ansprüchen 1 bis 12 für kosmetische und Toiletten-Zusammensetzungen.

## Revendications

1. Une composition polymère pulvérulente solide à étalement libre captive, émolliente et/ou humidifiante, dont les monomères ont été polymérisés sur place, caractérisée en ce qu'elle comprend de 5 à 95 pour cent en poids d'une grille polymère hydrophobe à chaine transversale, exempte de synérèse, dans laquelle sont incorporés comme émollient et/ou humidifiant 95 à 5 pour cent en poids· d'un composé choisi à partir d'un alcool droit, ramifié ou cyclique contenant de 1 à 30 atomes de carbone, d'un acide carboxylique droit, ramifié ou cyclique contenant de 1 à 30 atomes de carbone, d'un ester contenant un acide carboxylique $C_1$ à $C_{30}$ estérifié avec un alcool $C_1$ à $C_{30}$, d'un éther d'alcool contenant de 1 à 30 atomes de carbone, d'un alkane de la formule $H-(CH_2)_n-H$ dans laquelle n est de 5 à 30, de lanoline et de ses dérivés, et d'un siloxane.

2. La composition solide captive émolliente humidifiante selon la revendication 1, caractérisée en que ladite matrice polymère hydrophobe à chaîne transversale exempte de synérèse est basée sur un monomère à chaîne transversale di ou polyfonctionnel et sur un monomère mono- nonsaturé hydrophobe.

3. La composition solide captive émolliente et/ou humifiante selon la revendication 2, caractérisée en ce que ledit monomère à chaîne transversale est un monomère polynonsaturé choisi à partir d'un monoester, d'un diester ou d'un polyester d'alcool monohydrique, di- hydrique ou polyhydrique et d'un acide carboxylique non saturé alpha-béta, d'éther de polyvinyle polynonsaturé d'un alcool polyhydrique, d'amides monononsaturées ou polynonsaturées, et l'esters cycloaliphatiques d'acides carboxyliques non saturés alpha-béta.

4. La composition émolliente et/ou humidifiante solide captive selon la revendication 1, caractérisée en ce que ledit émollient et/ou humidifiant contient 10 à 90 pour cent en poids de parfum.

5. La composition solide captive émolliente et/ou humidifiante selon la revendication 1, caractérisée en ce que ledit émollient humidificant contient 0,1 à 10 pour cent en poids d'une matière colorante soluble dans l'huile.

6. La composition solide captive émolliente et/ou humidifiante selon la revendication 1, caractérisée en ce que ledit émollient-humidificant contient de 0,1 à 10 pour cent en poids d'une laque.

7. La composition solide captive émolliente et/ou humidifiante selon la revendication 1, caractérisée en ce que ledit émollient-humidifiant contient de 0,1 à 10 pour cent en poids d'un pigment.

8. La composition solide captive émolliente et/ou humidifiante selon la revendication 1, caractérisé en ce que ledit émollient-humidifiant est de l'oxystéarate 2-éthylhexylique.

9. La composition solide captive émolliente et/ou humidifiante selon la revendication 1, caractérisée en ce que ledit émollient-humidifiant est une huile minérale.

10. La composition solide captive émolliente et/ou humidifiante selon la revendication 1, caractérisée en ce que ledit émollient-humidificant est du propionate arachidylique.

11. La composition solide captive émolliente et/ou humidifiante selon la revendication 1, caractérisée en ce que ledit émollient-humidifiant est une bouillie de pétrole.

12. La composition solide captive émolliente et/ou humidifiante selon la revendication 1, caractérisée en ce que ledit émollient-humidifiant est un siloxane.

13. L'utilisation de la composition selon les revendications 1—12 pour une composition cosmétique ou une composition de toilette.